# EUROPÄISCHE PATENTANMELDUNG

(11) **EP 2 135 589 A1**
(43) Veröffentlichungstag der Anmeldung: **23.12.2009**
(21) Anmeldenummer: 09160157.5
(22) Anmeldetag: 13.05.2009
(51) Int. Cl.: A61F 2/84

(54) **Einführsystem für eine medizinische Einrichtung mit verbesserter Spülungsmöglichkeit**

(30) Priorität: 17.06.2008 DE 102008002472
(71) Anmelder: Biotronik VI Patent AG, 6341 Baar (CH)
(72) Erfinder: Moehl, Raimund, 8127, Forch (CH); Fargahi, Amir, 5242, Birr (CH)
(74) Vertreter: Lindner-Vogt, Karin L.

(57) **Zusammenfassung**

Die Erfindung betrifft ein Einführsystem (10) für eine medizinische Einrichtung (44) und ein Verfahren zum Betreiben eines Einführsystems für eine medizinische Einrichtung (44), insbesondere einen Stent, mit einem proximalen Ende (12) und einem distalen Ende (14), einem Innenschaft (30), der ein Innenlumen (32) einschließt und einem den Innenschaft (30) wenigstens bereichsweise umgebenden Außenschaft (40), der ein Zwischenlumen (42) umschließt. Eine oder mehrere Öffnungen (34, 36) sind im Innenschaft (30) ausgebildet, die eine Fluidverbindung zwischen dem Innenlumen (32) und dem Zwischenlumen (42) herstellen.

## Beschreibung

Die Erfindung betrifft ein Einführsystem für eine medizinische Einrichtung und ein Verfahren zum Betreiben eines Einführsystems für eine medizinische Einrichtung nach den Oberbegriffen der unabhängigen Ansprüche.

Es sind Einführsysteme für medizinische Einrichtungen wie etwa Gefäß-Stützkörper (Stents) bekannt. Zur Vermeidung eines Lufteintrags in das Gefäßsystem werden die Lumina eines Stent-Einführsystems vor dem Einführen mit einer Kochsalzlösung gespült und entlüftet. So werden etwa das Innenlumen, in dem der Führungsdraht geführt ist, oder das Zwischenlumen zwischen Innen- und Außenschaft, in dem der Stent während des Einführens angeordnet ist, gespült und entlüftet. Bei einigen bekannten Einführsystemen kann das Zwischenlumen nicht entlüftet werden. Aus der US 2007/0282420 A1 ist ein Stent-Einführsystem bekannt, das Entlüftungs- und Spülöffnungen im Außenschaft aufweist, welcher den Stent beim Einführen umgibt.

Bekannt sind andere Einführsysteme, bei denen das Führungsdrahtlumen und das Zwischenlumen von zwei separaten Anschlussstücken aus in gesonderten Arbeitsgängen entlüftet werden kann. Dazu sind jeweils separate T-Körper vorgesehen, durch die eine Spülflüssigkeit zugeführt und das jeweilige Lumen entlüftet werden kann.

Der Erfindung liegt die Aufgabe zugrunde, ein Einführsystem und ein Verfahren zum Betreiben eines solchen zu schaffen, mit dem eine einfache Handhabung und ein unkomplizierter Aufbau des Einführsystems möglich sind.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird ein Verfahren zum Betreiben eines Einführsystems für eine medizinische Einrichtung sowie ein Einführsystem für eine medizinische Einrichtung, insbesondere einen Stent, vorgeschlagen, mit einem proximalen Ende und einem distalen Ende, einem Innenschaft, der ein Innenlumen einschließt und einem den Innenschaft wenigstens bereichsweise umgebenden Außenschaft, der ein Zwischenlumen umschließt. Eine oder mehrere Öffnungen sind im Innenschaft ausgebildet, die eine Fluidverbindung zwischen dem Innenlumen und dem Zwischenlumen herstellen. Dadurch kann das Spülen und/oder Entlüften des Innenlumens und des Zwischenlumens mit einer einzigen Vorrichtung erfolgen. Zum einen ist das Innenlumen zugänglich, zum anderen sind keine getrennten Anschlüsse zum Spülen und/oder Entlüften des Innenlumens und des Zwischenlumens notwendig. Der Aufbau der Einführvorrichtung kann dadurch vereinfacht werden.

Kann eine erste Öffnung benachbart zu einem proximalen Ende und eine weitere Öffnung an einem distalen Ende des Innenschafts angeordnet sein, kann das jeweilige Lumen entlang seiner Längserstreckung zuverlässig gespült und entlüftet werden. Dabei kann an dem proximalen Ende eine Koppelvorrichtung zum Spülen und/oder Entlüften des Innenlumens und des Zwischenlumens mit einem Fluid vorgesehen sein. Diese Anordnung erlaubt einen handlichen, kompakten Aufbau der Einführvorrichtung. Bevorzugt kann die Koppelvorrichtung dabei an einem Anschlussstück des Innenlumens angeordnet sein.

Gemäß dem vorgeschlagenen Verfahren zum Betreiben eines Einführsystems für eine medizinische Einrichtung wird ein Spülen und/oder Entlüften des Innenlumens und des Zwischenlumens in einem einzigen Arbeitsgang durchgeführt. Die Handhabung des Einführsystems wird dadurch vereinfacht.

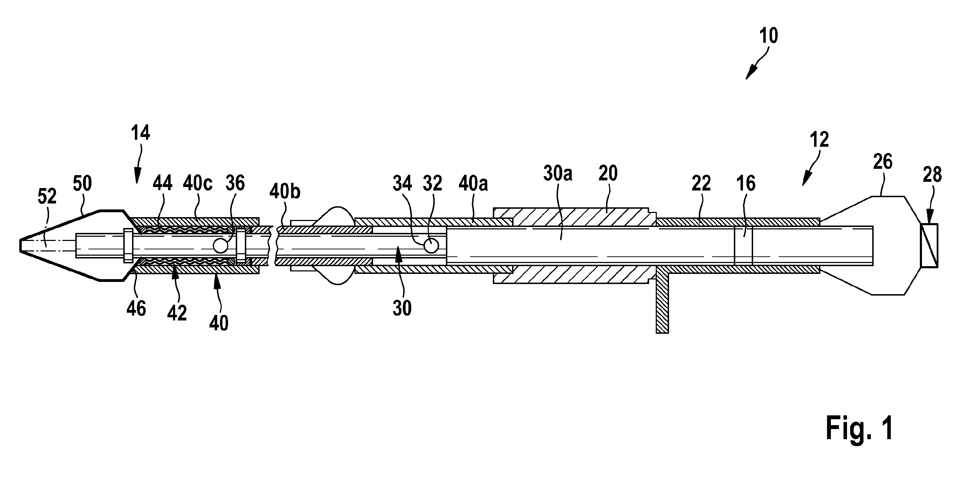

Die Erfindung ist nachfolgend beispielhaft anhand von in Zeichnungen dargestellten Ausführungsbeispielen näher erläutert. Es zeigt die einzige Figur in schematischer Darstellung einen Längsschnitt durch ein bevorzugtes Einführsystem mit einem Stent.

Zur Erläuterung der Erfindung zeigt die Figur einen Längsschnitt durch ein beispielhaftes bevorzugtes Einführsystem 10 für eine medizinische Einrichtung 44 gemäß der Erfindung. Die medizinische Einrichtung 44 kann z.B. ein Stent, insbesondere ein selbstexpandierender Stent sein.

Das Einführungssystem 10 weist ein proximales Ende 12 und ein distales Ende 14 auf, zwischen denen sich ein Innenschaft 30 erstreckt. Der Innenschaft 30 schließt ein Innenlumen 32 ein, in dem ein Führungsdraht (nicht dargestellt) geführt ist, der das Einführen der als Stent ausgebildeten medizinischen Einrichtung 44 erleichtert. Am proximalen Ende 12 ist ein Griffstück 20 angeordnet, das als Haltegriff für das Einführsystem dient.

Ein Außenschaft 40 umgibt den Innenschaft 30 wenigstens bereichsweise. Der Außenschaft 40 umschließt dabei ein Zwischenlumen 42 zwischen der Außenseite des Innenschafts 30 und der Innenseite des Außenschafts 40. Der Außenschaft 40 reicht nur bis zum Haltegriff 20 und ist mehrteilig ausgebildet. Der Außenschaft 40 besteht aus den Teilstücken 40a, 40b, 40c. Das distale Teilstück 40c umschließt beim Einführen die medizinische Einrichtung 44, das mittlere Teilstück 40b umschließt einen mittleren Bereich des Innenschafts 30 relativ eng, und das proximale Teilstück 40a stößt an das Griffstück 20 an. Das Zwischenlumen 42 hat entsprechend den Innendurchmessern der Teilstücke 40a, 40b, 40c des Außenschafts 40 jeweils eine unterschiedliche radiale Abmessung entlang dem Innenschaft 30.

Die als Stent ausgebildete medizinische Einrichtung 44 befindet sich innerhalb des dritten Teilstücks 40c des Außenschafts 40 und ist im Zwischenlumen 42 mit üblichen Haltemitteln und dergleichen in komprimierter Form am distalen Ende 14 gelagert. Am distalen Ende 14 ist ferner eine Spitze 50 angeordnet, in welcher ein Führungsdraht (nicht dargestellt) in einem Drahtlumen 52 geführt ist, welches durchgängig von der Spitze 50 bis zu einem Zugang 28 am proximalen Ende 12 reicht. Beim Einführvorgang ragt der Führungsdraht aus dem freien Ende der Koppelvorrichtung 26 heraus.

Mit Hilfe des Griffstücks 20 am proximalen Ende 12 kann der Außenschaft 40 zurückgezogen werden, um die als Stent ausgebildete medizinische Einrichtung 44 freizusetzen. Eine zum Freisetzen entfernbare Sicherungshülse 22 zum Sichern der relativen Position von Innenschaft 30 zu Außenschaft 40 ist zwischen dem Griffstück 20 und dem proximalen Ende 12 angeordnet. Ein Markierungselement 16 dient beim Freisetzen der medizinischen Einrichtung 44 zur Anzeige, ob die medizinische Einrichtung 44 ganz freigesetzt ist oder nicht.

Der Innenschaft 30 ist am proximalen Ende 12 zur besseren Handhabung z.B. durch einen Metallschaft 30a gebildet, auf dem das Markierungselement 16 angeordnet ist.

Am proximalen Ende 12 ist an das Griffstück 20 eine Koppelvorrichtung 26 angesetzt, die einen Zugang 28 aufweist, durch den das Innenlumen 32 und das Zwischenlumen 42 mit einem Fluid, z.B. einer Kochsalzlösung, gespült bzw. entlüftet werden kann. Zwischen dem Griffstück 20 und der Koppelvorrichtung 26 ist die Sicherungshülse 22 angeordnet.

Am proximalen Ende des Haltegriffs 20 ist eine Dichtung vorgesehen (nicht dargestellt), damit das Fluid zum Spülen nicht proximal entweichen kann, sondern nur am distalen Ende 14.

Der Innenschaft 30 weist im Bereich des ersten Teilstücks 40a des Außenschafts 40 benachbart zum proximalen Ende 12 mindestens eine erste Öffnung 34 auf und am distalen Ende 14 mindestens eine zweite Öffnung 36, so dass zwischen dem Innenlumen 32 und dem Zwischenlumen 42 eine Fluidverbindung ausgebildet ist. Das Fluid wird über den Zugang 28 der Koppelvorrichtung 26 in das Innenlumen 32 des Innenschafts 30 zugeführt und kann durch die Öffnungen 34 und 36 in das Zwischenlumen 42 austreten. Das Fluid kann am distalen Ende am Übergang 46 zwischen dem dritten Teilstück 40c des Außenschafts 40 und der Spitze 50 aus dem Zwischenlumen 42 nach außen austreten, da der Außenschaft 40 nicht dichtend an die Spitze 50 anstößt.

Das Spülen bzw. Entlüften des Innenlumens 32 und des Zwischenlumens 42 kann vorteilhaft in einem einzigen Arbeitsgang vom Koppelstück 26 aus erfolgen.

## Patentansprüche

1. Einführsystem (10) für eine medizinische Einrichtung (44), insbesondere einen Stent, mit einem proximalen Ende (12) und einem distalen Ende (14), einem Innenschaft (30), der ein Innenlumen (32) einschließt und einem den Innenschaft (30) wenigstens bereichsweise umgebenden Außenschaft (40), der ein Zwischenlumen (42) umschließt, **dadurch gekennzeichnet, dass** eine oder mehrere Öffnungen (34, 36) im Innenschaft (30) ausgebildet sind, die eine Fluidverbindung zwischen dem Innenlumen (32) und dem Zwischenlumen (42) herstellen.

2. Einführsystem nach Anspruch 1, **dadurch gekennzeichnet, dass** eine erste Öffnung (34) benachbart zum proximalen Ende (12) und eine weitere Öffnung (36) an einem distalen Ende (14) des Innenschafts (30) angeordnet ist.

3. Einführsystem nach Anspruch 1 oder 2, **dadurch gekennzeichnet, dass** an dem proximalen Ende (12) eine Koppelvorrichtung (26) zum Spülen und/oder Entlüften eines Fluids des Innenlumens (32) und des Zwischenlumens (42) angeordnet ist.

4. Einführsystem nach Anspruch 3, **dadurch gekennzeichnet, dass** die Koppelvorrichtung (26) an einem Griffstück (20) des Innenlumens (32) angeordnet ist.

5. Verfahren zum Betreiben eines Einführsystems (10) für eine medizinische Einrichtung (44), nach einem der vorhergehenden Ansprüche, insbesondere zum einführen eines Stents, mit proximalen Ende (12) und einem distalen Ende (14), bei dem ein Innenschaft (30) ein Innenlumen (32) einschließt und ein Außenschaft (40), der den Innenschaft (30) wenigstens bereichsweise umgibt, ein Zwischenlumen (42) umschließt, **dadurch gekennzeichnet, dass** ein Spülen und/oder Entlüften des Innenlumens (32) und des Zwischenlumens (42) in einem einzigen Arbeitsgang durchgeführt wird
